# EUROPEAN PATENT SPECIFICATION

(11) **EP 0 292 951 B1**
(45) Date of publication and mention of the grant of the patent: **16.03.1994**
(21) Application number: 88108368.7
(22) Date of filing: 26.05.1988
(51) Int. Cl.: C07C 37/86, C07C 39/07

(54) **Process for purifying 2,4-xylenol**
Verfahren zur Reinigung von 2,4-Xylenol
Procédé de purification de xylénol-2,4

(30) Priority: 29.05.1987 JP 131694/87; 12.05.1988 JP 115119/88
(43) Date of publication of application: 30.11.1988
(73) Proprietor: MITSUI TOATSU CHEMICALS, Inc., Chiyoda-Ku Tokyo 100 (JP)
(72) Inventor: Miura, Tohru, Omuta-shi Fukuoka (JP); Watanabe, Katsuji, Omuta-shi Fukuoka (JP); Nakayama, Hitoshi, Omuta-shi Fukuoka (JP); Nagata, Teruyuki, Omuta-shi Fukuoka (JP); Furuya, Masayuki, Omuta-shi Fukuoka (JP)
(74) Representative: Gervasi, Gemma, Dr.

(56) References cited:
- US-A- 2 917 487
- PATENT ABSTRACTS OF JAPAN, vol. 103, no. 77, (C-392) 16th December 1986; & JP-A-61 168 611
- PATENT ABSTRACTS OF JAPAN, vol. 12, no. 47 (C-475)[2894], 12th February 1988; & JP-A-62 192 331

## Description

The present invention concerns a method for purifying a crude 2,4-xylenol mixture containing 2,5-xylenol. More specifically, the invention concerns a method for obtaining 2,4-xylenol with high purity by separating 2,5-xylenol from a crude 2,4-xylenol isomer mixture containing 2,5-xylenol.

2,4-xylenol is a compound useful as the starting material for synthetic resins, adhesives, insecticides, antioxidants, herbicides, dyes, etc. and has been produced and used in a great amount. Depending on the case, products of high purity are required.

### Description of the Prior Art

Xylenol includes six isomers, that is, 2,3-xylenol, 2,4-xylenol, 2,5-xylenol, 2,6-xylenol, 3,4-xylenol and 3,5-xylenol. Since boiling points of these isomers are closed to each other, it is extremely difficult to efficiently separate the respective isomer component from the xylenol isomer mixture by means of distillation. Particularly, since the boiling points of 2,4-xylenol and 2,5-xylenol are substantially identical, they can not be separated by mere rectification. In view of the above, 2,4-xylenol with the purity of about 90 - 95 % at the highest is commercially available at present.

There have been made various proposals for the method of separating 2,4-xylenol and 2,5-xylenol. For instance, there have been proposed a separating and purifying method by utilizing the nature of 2,4-xylenol that forms a molecular compound with methyl quinoline, aniline, toluidine, etc. (for example, refer to U.S. Patent No. 2,526,807), a separating and purifying method by alkylation into tert-butylated product by isobutylene, separating the tert-butylated product by fractional distillation and then carrying out dealkylation in the presence of an acid (for example, refer to British Patent No. 582,057), a separating method by sulfonation and, successively, carrying out partial hydrolysis with super heated steams (for example, refer to U.S. Patent No. 2,327,312), as well as a method of using adsorbents such as molecular sieves, calcium oxide or zeolite.

Furthermore, there has also been proposed a method of reacting xylenol with formalin in the presence of a strong acid such as hydrochloric acid or a strong base such as sodium hydroxide and then separating only the 2,5-xylenol in the form of a xylenol-formaline resin (for example, refer to U.S. Patent No. 2,917,487).

U.S. Patent No. 2,917,487 discloses conducting a condensation reaction at temperatures of 50° - 100°C with use of of hydrochloric acid as a catalyst in the amount of 2 - 8 % by weight to obtain purified 2,4-xylenol with purity of 95.1 % at the highest and purity-converted yield of 67.3 %. However, both of such purity and yield are not quite satisfactory. Particularly, depending on uses of 2,4-xylenol, purification products of a purity higher than 95 % and, desirably, from 98 to 99 % may some time be demanded. Purification products of such a high purity can not be obtained by the method of the above-mentioned U.S. Patent without reducing the yield of 2,4-xylenol. In addition, according to this method, after selectively condensating only the 2,5-xylenol with formalin in the presence of the acid catalyst, the reaction mass is distilled under heating for separating 2,4-xylenol and the acid used for the condensation reaction is removed by water washing. However, the removal of hydrochloric acid with water washing causes a problem of treatment of the waste water in an industrial practice. The problem may be overcome by removing hydrochloric acid through neutralization by adding an alkali such as sodium hydroxide to the reaction mass after the condensation reaction. However, in this case, since the reaction mass containing the alkali metal is heated, a care must be taken in view of the handling safety. Further, the loss in 2,4-xylenol is innegligible.

In this way, it is very difficult to industrially obtain 2,4-xylenol with high purity, which is a problem that has not yet been solved.

### SUMMARY OF THE INVENTION

An object of the present invention is to provide an industrial purification method of 2,4-xylenol by separating 2,5-xylenol from a crude 2,4-xylenol mixture including 2,5-xylenol.

The second object of the present invention is to provide an improved purification method capable of obtaining 2,4-xylenol with higher purity without reducing the yield upon separating 2,5-xylenol.

The foregoing object of the present invention can be attained by reacting a crude 2,4-xylenol mixture containing 2,5-xylenol with a specific amount of aldehyde in the present of an aromatic sulfonic acid and separating 2,5-xylenol in the form of a xylenol-aldehyde condensate from 2,4-xylenol.

The present inventors have diligently studied the purification method of 2,4-xylenol of removing 2,5-xylenol contained in a crude 2,4-xylenol isomer mixture, and have found that when the crude 2,4-xylenol is reacted with an aldehyde in the presence of an aromatic sulfonic acid selected as an acid catalyst from strong acids, 2,5-xylenol can preferentially be reacted with the aldehyde at a relatively low temperature.

Accordingly, in the method of the present invention, a specific amount of an aldehyde added to the reaction system selectively reacts with 2,5-xylenol in the crude 2,4 xylenol mixture substantially completely, whereas it is scarcely consumed in the reaction with 2,4-xylenol and, accordingly, 2,4-xylenol with high purity of higher than 95 % can be obtained without separation loss of 2,4-xylenol.

Furthermore, if a specific amount of water is present together in the reaction system, 2,4-xylenol can be obtained with a higher purity and higher yield. That is, 2,4-xylenol can be obtained with high purity and high yield when conducting the reaction with the aldehyde while adding water in such an amount as not exceeding the saturation solubility based on the entire amount of xylenol to be processed into the reaction system together with the aromatic sulfonic acid. In this case, the reaction with the aldehyde can be conducted in a wide range of temperature by properly selecting the amount of water added. The present invention has thus been completed based on these findings.

In the method according to the present invention, only the 2,5-xylenol is reacted with an aldehyde selectively in the presence of an aromatic sulfonic acid catalyst, the acid catalyst remaining in the reaction mass is then removed by a conventional method and, thereafter, the reaction mass is subjected to distillation, etc. to recover 2,4-xylenol as the distillation solution, while separating 2,5-xylenol-aldehyde condensates as the still residues. In this way, 2,4-xylenol of high purity can be obtained with high yield.

### DETAILED DESCRIPTION OF THE INVENTION

In the method according to the present invention, there is no particular restriction for the composition of the crude 2,4-xylenol as the xylenol isomer mixture to be used, but the method of the present invention has a particularly remarkable effect for the purification of 2,4-xylenol to purify 2,4-xylenol from 80 - 95 % purity to 95 % or higher purity. In many cases, crude xylenols containing 80 - 95 % of 2,4-xylenol are used as the starting material. Furthermore, other xylenol isomers such as 2,3-xylenol can also be separated through condensation reaction with the aldehyde in the same manner as that for 2,5-xylenol, whereas those alkylphenols less reactive with the aldehyde such as 2,4,6-trimethylphenol can not be removed by the method according to the present invention. Accordingly, in a case a great amount of alkyl phenols is contained, other separation methods, for example, distillation or recrystallization may preferably be used in combination with the method according to the present invention.

The aldehyde compound used for the condensation in the present invention can include, for example, low molecular weight aliphatic aldehydes such as formaldehyde, acetoaldehyde, propion aldehyde and butyl aldehyde, as well as aromatic aldehydes such as benzaldehyde. Among these aldehydes, formaldehyde and acetoaldehyde are preferred in view of the reactivity, selectivity, cost, yield and usage of condensates. In this case, the aldehyde may be used in any of forms such as aqueous solution, alcohol solution, trimer, polymer or acetal. In the case of using the aldehyde in hydrous form such as an aqueous solution, the amount of water present in the reaction system is controlled in accordance with the amount of water introduced by aldehyde used into the reaction system, where the amount of water in the condensation reaction system is less than the saturation solubility based on the entire amount of xylenol mixture, that is, so that it does not form the dual layer of crude 2,4-xylenol solution and water in the reaction system.

Further, the amount of the aldehyde used differs more or less depending on the amount of 2,5-xylenol to be removed, reaction conditions, etc. but it is desirable to select the reaction conditions such that 2,4-xylenol with high purity can be obtained by using the aldehyde in an amount as less as possible. Usually, the aldehyde is used by from 0.3 to 3 molar times, preferably, from 0.3 to 2.0 molar times of 2,5-xylenol contained and the reaction is continued till the aldehyde content is consumed entirely.

In the condensation reaction of the present invention, the aromatic sulfonic acid used as the catalyst can include, for example, benzene sulfonic acid, ortho-, metha- and paratoluene sulfonic acid and dibenzene sulfonic acid. Particularly, para-toluene sulfonic acid is most preferred catalyst.

The amount of the catalyst used is usually within a range from 0.001 to 10 parts by weight and, preferably, from 0.01 to 1 parts by weight based on 100 parts by weight of the crude 2,4-xylenol. If the amount of the catalyst used is excessive, the purity of the xylenol obtained is lowered.

The reaction temperature in the present invention can be controlled by the amount of water added and the reaction can be carried out within a wide temperature range from 0 to 100°C and, preferably, from 20 to 60°C. However, since the catalyst according to the present invention can provide the effect at a relatively low temperature, the reaction is preferably practiced at a temperature lower than 50°C in the case not adding water. Higher reaction temperature is not preferred since 2,4-xylenol is also brought into reaction to cause loss by so much and the purity of the 2,4-xylenol is lowered. Lower reaction temperature is neither advantageous since the reaction speed is reduced to take an extremely long reaction time.

In the case of practicing the condensation reaction according to the present invention in the presence of water, the amount of water added is such that it does not exceed the saturation solubility of the xylenol isomer mixture at the temperature of conducting the reaction. Specifically, water is added in such an amount that preferably from 2 to 20 parts by weight of water is present in the reaction system based on 100 parts by weight of the xylenol isomer mixture. Since it is usually desired to practice the reaction at the temperature from 20 to 60°C, the solubility of the entire xylenol isomer mixture in water in this case is about from 5 to 20 % and water is added by an amount not exceeding this range. If water is added exceeding the solubility, since the reaction is conducted under the state separated to two layers and the reaction speed is extremely lowered, it is not advantageous.

In addition, since the xylenol isomer mixture shows various coagulation points depending on the composition, in a case the xylenol isomer mixture as the starting material is solid within the temperature range for the condensation reaction, a solvent not giving undesired effect to the reaction may be used. The solvent suitable for such a case can include, for example, aromatic hydrocarbons such as benzene, toluene, xylene, cumene and mesitylene; aliphatic hydrocarbons such as hexane, heptane and octane; halogenated hydrocarbons such as chlorobenzene, ortho-dichlorobenzene, chloroform, carbon tetrachloride and dichloroethane; etc.

When the purity and yield of purified 2,4-xylenol, recovery rate, cost, performance of the solvent, etc. are taken into consideration, toluene is one of the most preferred solvents. In a case the starting xylenol isomer mixture is liquid at the reaction temperature, the reaction may be carried out while using the solvent as described above or without using such a solvent.

In the case of using the solvent, the amount is usually less than 500 parts by weight and, preferably, less than 200 parts by weight based on the 100 parts by weight of the crude 2,4-xylenol.

Since the dehydration-condensation reaction between xylenol and aldehyde is an equilibrium reaction, if the reaction mass is directly subjected to distillation, 2,5-xylenol-aldehyde condensates formed in the reaction step undergo hydrolysis during distillation into 2,5-xylenol, which may intrude into the distillation solution thereby to reduce the purity of 2,4-xylenol. Accordingly, the acid used as the catalyst as described above has to be removed before distillation and the aromatic sulfonic acid is removed before distillation also in the present invention.

Aromatic sulfonic acid can be removed by a method of water washing and liquid separation of the reaction mass after the end of the condensation reaction or neutralization with a base. However, since even the acid removal with the neutralization gives no substantial effect on the purity of the purification product in the present invention, it is preferred for the method of taking out 2,4-xylenol in the present invention to neutralize the reaction products with an excess amount of aqueous ammonia after the completion of the reaction and then apply distillation. In the purification method using hydrochloric acid as the catalyst in the reference as described above, since ammonium chloride formed upon neutralization with aqueous ammonia sublimates during distillation and intrudes into 2,4-xylenol, such a method can not be used. The method according to the invention can be said extremely advantageous also from this point of view.

As has been described above, in the present invention, pure 2,4-xylenol can be obtained as the distillation solution by applying distillation after neutralization with aqueous ammonia. After the neutralization and distillation, xylenol-aldehyde condensates and ammonium salt of aromatic sulfonic acid remain in the distillation can, but the condensates can be purified and recovered, as required, by way of conventional method such as water washing, recrystallization, etc. for the ammonium salt of aromatic sulfonic acid.

The present invention will be described more specifically referring to examples and comparative examples.

### Example 1

To a 500 mℓ volume four-necked flask made of glass, 244.4 g of crude 2,4-xylenol of the composition shown in Table 1, 6.6 g of 90 % para-formaldehyde and 0.2 g of p-toluene sulfonic acid were charged and reacted at a reaction temperature of 20°C for 4 hours while stirring. Then, after neutralization with addition of 0.1 mℓ of an aqueous 25 % ammonia solution, distillation under a reduced pressure was applied to obtain 196.1 g of fractions boiling at 104°C/21 mmHg (pure 2,4-xylenol). The composition was shown in Table 1. The purity-converted recovery yield of 2,4-xylenol was 86.3 %.

### Example 2

In the same procedures as in Example 1 except for changing the reaction temperature to 80°C and reaction time to one hour to obtain 195.0 g of purified 2,4-xylenol of the composition shown in Table 1.

### Example 3

In the same procedures as in Example 1 except for changing the reaction temperature to 0°C and reaction time to 20 hours to obtain 195.3 g of purified 2,4-xylenol of the composition shown in Table 1.

### Example 4

In the same procedures as in Example 1 except for changing 6.6 g of 90 % p-formaldehyde to 17.2 g of an aqueous 35 % formalin solution to obtain 193.6 g of purified 2,4-xylenol of the composition shown in Table 1.

### Example 5

In the same procedures as in Example 1 except for changing 6.6 g of 90 % p-formaldehyde to 9.7 g of an aqueous 90 % acetoaldehyde solution to obtain 194.9 g of purified 2,4-xylenol of the composition shown in Table 1.

### Example 6

In the same procedures as in Example 1 except for replacing 0.2 g of p-toluene sulfonic acid to 0.2 g of benzene sulfonic acid to obtain 195.7 g of purified 2,4-xylenol of the composition shown in Table 1.

### Example 7

To a 500 mℓ volume four-necked flask made of glass, 244.4 g of crude 2,4-xylenol of the composition shown in Table 1, 6.6 g of 90 % para-formaldehyde, 0.2 g of p-toluene sulfonic acid and 200 mℓ of toluene were charged and reacted at a reaction temperature of 0°C for 10 hours. After the reaction was over, 0.2 g of an aqueous 25 % ammonia and 50 mℓ of water were added under cooling, and after stirring for one hour, they were subjected to liquid separation. Then, after washing with 50 mℓ of water, toluene was removed by an evaporator. The residue was distilled under a reduced pressure to obtain 190.1 g of fractions boiling at 104°C/21 mmHg (purified 2,4-xylenol). The composition of purified 2,4-xylenol is shown in Table 1.

### Example 8

To a 500 mℓ volume four-necked flask made of glass, 244.4 g of crude 2,4-xylenol of the composition shown in Table 1, 17.2 g of 35 % aqueous-formalin solution and 0.1 g of p-toluene sulfonic acid were charged and reacted at a reaction temperature of 20°C for 4 hours while stirring. After the reaction was over, 200 mℓ of water was added and stirred sufficiently and then the resultant was subject to liquid separation. The oil layer was distilled under a reduced pressure to obtain 188.3 g of fractions boiling at 104°C/21 mmHg (purified 2,4-xylenol). The composition of the purified 2,4-xylenol was shown in Table 1.

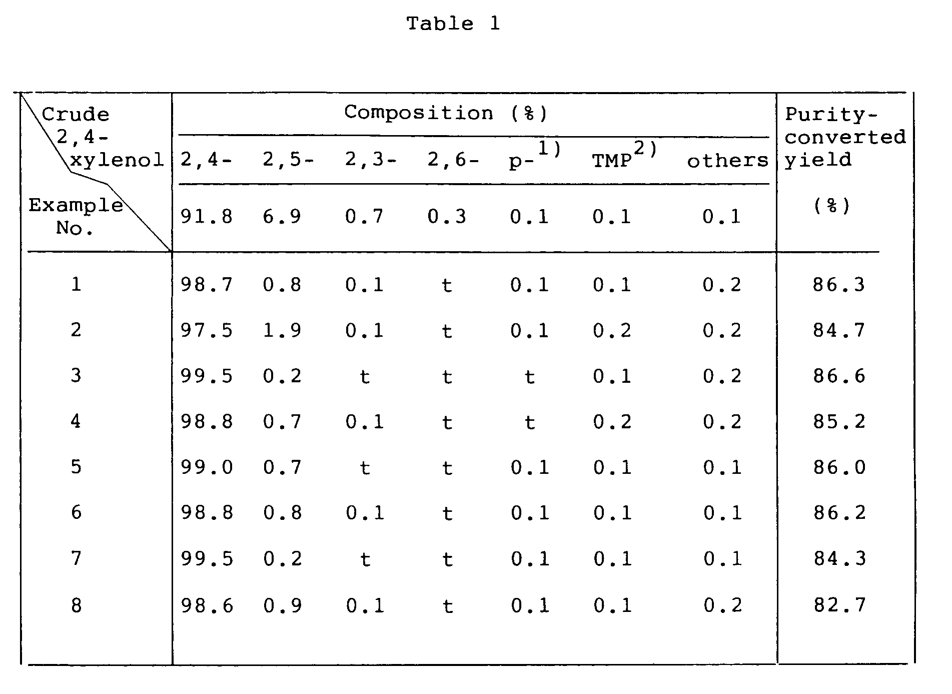

### Example 9

To a 500 mℓ volume four-necked flask made of glass, 244.4 g of crude 2,4-xylenol of the composition shown in Table 2, 19.3 g of 90 % para-formaldehyde and 0.1 g of p-toluene sulfonic acid were charged and reacted at a reaction temperature of 20°C for 5 hours. After the reaction was over, reaction product was neutralized with an aqueous 25 % ammonia and then distilled to obtain 175.6 g of purified 2,4-xylenol. The composition was shown in Table 2. The purity-converted recovery yield of 2,4-xylenol was 85 %.

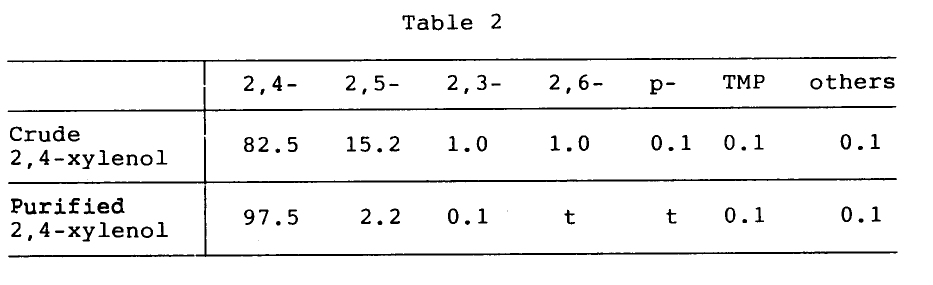

### Comparative Example 1

Reaction was conducted according to U.S. Patent No. 2,917,487. That is, to a 500 mℓ volume four-necked flask made of glass, 244.4 g of crude 2,4-xylenol of the composition shown in Table 1 and 6.6 g of 90 % para-formaldehyde were charged and kept at 100°C. Then 40 mℓ of 36 % hydrochloric acid was charged and reacted at 100°C for one hour. Then, after neutralization with addition of 30 mℓ of an aqueous 25 % ammonia, the product was distilled to obtain 194.8 g of fractions boiling at 104°C/21 mmHg. The fractions containing 400 ppm of ammonium chloride were opaque and the 2,4-xylenol purity was 94.9 %. Main impurity was 2,5-xylenol.

### Example 10

To a 500 mℓ volume four-necked flask made of glass, 244.4 g of crude 2,4-xylenol of the composition shown in Table 1, 6.6 g of 90 % para-formaldehyde, 0.2 g of p-toluene sulfonic acid, 200 mℓ of toluene and 20 g of water were charged and reacted at a reaction temperature of 40°C for 10 hours. After the reaction was over, the reaction product was neutralized with 1 mℓ of an aqueous 25 % ammonia and then distilled to obtain 194.5 g of pure 2,4-xylenol fractions boiling at 104°C/21 mmHg (purified 2,4-xylenol). The composition was shown in Table 3. The purity-converted recovery yield of 2,4-xylenol was 85.9 %.

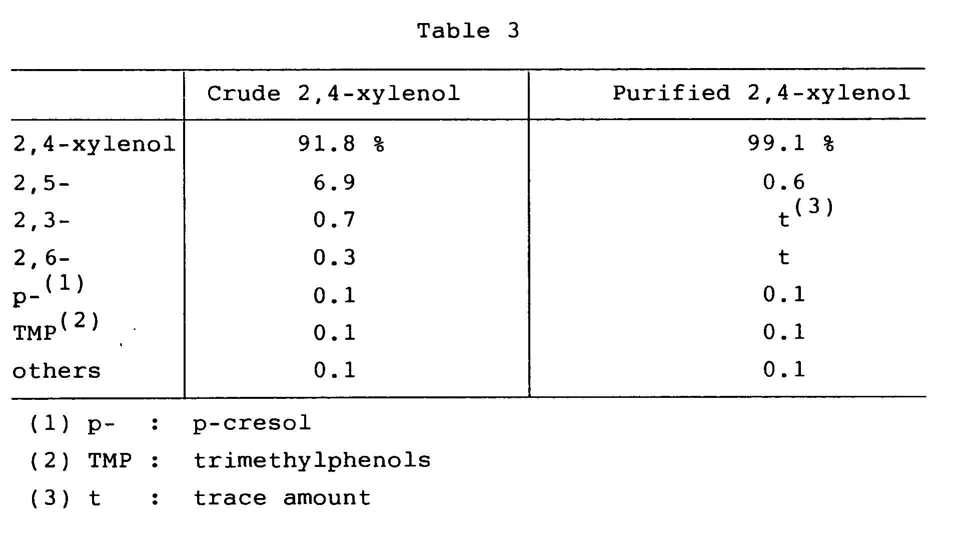

### Comparative Example 2

Reaction was conducted according to U.S. Patent No. 2,917,487. That is, to a 500 mℓ volume four-necked flask made of glass, 244.4 g of crude 2,4-xylenol of the composition shown in Table 1 and 6.6 g of 90 % para-formaldehyde were charged and kept at 100°C. Then 40 mℓ of 36 % hydrogen chloride was charged and reacted at 100°C for one hour. Then, after neutralization with addition of 30 mℓ of an aqueous 25 % ammonia, the product was distilled to obtain 194.8 g of fractions boiling at 104°C/21 mmHg. The fractions containing 400 ppm of ammonium chloride were opaque and the 2,4-xylenol purity was 94.9 %. Main impurity was 2,5-xylenol. The purity-converted recovery yield was 82.4 %.

### Example 11

To a 500 mℓ volume four-necked flask made of glass, 244.4 g of crude 2,4-xylenol of the composition shown in Table 2, 19.3 g of 90 % para-formaldehyde, 0.2 g of p-toluene sulfonic acid, 200 mℓ of toluene and 20 g of water were charged and reacted and processed in the same procedures as in Example 10 to obtain 173.3 g of pure 2,4-xylenol of the composition shown in Table 4. The purity-converted recovery yield of 2,4-xylenol was 85 %.

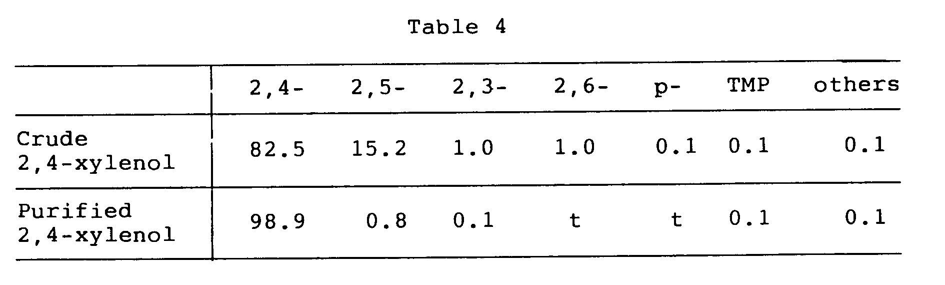

### Example 12

Reaction and processing were conducted in the same procedures as in Example 10 except for replacing 6.6 g of 90 % p-formaldehyde with 17.0 g of 35 % formalin and changing the amount of water added from 20 to 10 g to obtain 194.4 g of pure 2,4-xylenol. The composition was 99.2 % of 2,4-xylenol and 0.6 % of 2,5-isomer and the purity-converted yield was 85.9 %.

### Example 13

To a 500 mℓ volume four-necked flask made of glass 244.4 g of crude 2,4-xylenol of the composition shown in Table 1, 6.6 g of 90 % para-formaldehyde, 0.2 g of p-toluene sulfonic acid and 30 g of water were charged and reacted at a reaction temperature of 60°C for 10 hours. After the reaction was over, they were processed in the same procedures as in Example 10 to obtain 194.0 g of purified 2,4-xylenol. The composition was 98.8 % of 2,4-xylenol and 0.8 % of 2,5-isomer and the purity-converted yield was 85.4 %.

## Claims

1. A method of purifying 2,4-xylenol from a crude 2,4-xylenol isomer mixture containing 2,5-xylenol, which comprises reacting the crude 2,4-xylenol mixture with an aldehyde of from 0.3 to 3.0 molar times the amount of 2,5-xylenol in the presence of an aromatic sulfonic acid and then separating 2,5-xylenol as a xylenol-aldehyde condensate from 2,4-xylenol.

2. A method of purifying 2,4-xylenol from a crude 2,4-xylenol isomer mixture containing 2,5-xylenol, which comprises reacting the crude 2,4-xylenol mixture with an aldehyde of from 0.3 to 3.0 molar times the amount of 2,5-xylenol in the presence of an aromatic sulfonic acid while incorporating into the reaction system water in such an amount as not separating into a dual layer of water and the crude 2,4-xylenol and then separating 2,5-xylenol as a xylenol-aldehyde condensate from 2,4-xylenol.

3. A method according to claim 1 wherein the crude 2,4-xylenol isomer mixture used as starting material contains 2,4-xylenol in an amount comprised between 80% to 95%.

4. A method according to claim 1 wherein the reaction between aldehyde and 2,5-xylenol is performed at a temperature comprised between 0° - 100°C

5. A method according to claim 4 wherein the temperature is comprised between 20° - 60° C.

6. A method according to claim 1 wherein the amount of the aromatic sulfonic acid is from 0.001 - 10 parts by weight based on 100 parts by weight of the crude 2,4-xylenol.

7. A method according to claim 6 wherein the amount of the aromatic sulfonic acid is from 0.01 - 1 parts by weight based on 100 parts by weight of the crude 2,4-xylenol.

8. A method according to claim 2 wherein the amount of water incorporated into the reaction system is from 2 to 20 parts by weight based on 100 parts by weight of the crude 2,4-xylenol.

9. A method of purifying 2,4-xylenol from a crude 2,4-xylenol isomer mixture containing 2,5-xylenol, which comprises reacting the crude 2,4-xylenol mixture with an aldehyde of from 0.3 to 3.0 molar times the amount of 2,5-xylenol in the presence of an aromatic sulfonic acid, or in the presence of an aromatic sulfonic acid coexistent with water in such an amount as not causing a dual layer separation to water and 2,4-xylenol, converting 2,5-xylenol into a xylenol-aldehyde condensate and converting the aromatic sulfonic acid into an ammonium salt thereof after the end of the reaction and, therafter distilling reaction products to separate 2,4-xylenol by distillation.

## Patentansprüche

1. Verfahren zur Reinigung von 2,4-Xylenol aus rohem 2,4-Xylenol in Form eines 2,5-Xylenol enthaltenden Isomerengemisches, welches die Umsetzung des rohen 2,4-Xylenol-Gemisches mit einem Aldehyd in der 0,3- bis 3,0-fachen molaren Menge des 2,5-Xylenols in Gegenwart einer aromatischen Sulfonsäure und die anschließende Abtrennung des 2,5-Xylenols in Form eines Xylenol-Aldehyd-Kondensationsprodukts von 2,4-Xylenol umfaßt.

2. Verfahren zur Reinigung von 2,4-Xylenol aus rohem 2,4-Xylenol in Form eines 2,5-Xylenol enthaltenden Isomerengemisches, welches die Umsetzung des rohen 2,4-Xylenol-Gemisches mit einem Aldehyd in der 0,3- bis 3,0-fachen molaren Menge des 2,5-Xylenols in Gegenwart einer aromatischen Sulfonsäure unter Zugabe von Wasser zum Reaktionssystem in einer solchen Menge, daß die Trennung in zwei Schichten aus Wasser und dem rohen 2,4-Xylenol unterbleibt, und die anschließende Abtrennung des 2,5-Xylenols als Xylenol-Aldehyd-Kondensationsprodukt von 2,4-Xylenol umfaßt.

3. Verfahren gemäß Anspruch 1, worin das als Ausgangsmaterial verwendete rohe 2,4-Xylenol in Form eines Isomerengemisches 2,4-Xylenol in einer Menge von 80 bis 95 % enthält.

4. Verfahren gemäß Anspruch 1, worin die Umsetzung zwischen dem Aldehyd und 2,5-Xylenol bei einer Temperatur im Bereich von 0 bis 100°C durchgeführt wird.

5. Verfahren gemäß Anspruch 4, worin die Temperatur im Bereich von 20 bis 60°C liegt.

6. Verfahren gemäß Anspruch 1, worin die Menge der aromatischen Sulfonsäure 0,001 bis 10 Gew.-Teile, bezogen auf 100 Gew.-Teile des rohen 2,4-Xylenols beträgt.

7. Verfahren gemäß Anspruch 6, worin die Menge der aromatischen Sulfonsäure 0,01 bis 1 Gew.-Teil, bezogen auf 100 Gew.-Teile des rohen 2,4-Xylenols beträgt.

8. Verfahren gemäß Anspruch 2, worin die Menge des zum Reaktionssystem zugegebenen Wassers 2 bis 20 Gew.-Teile, bezogen auf 100 Gew.-Teile des rohen 2,4-Xylenols beträgt.

9. Verfahren zur Reinigung von 2,4-Xylenol aus rohem 2,4-Xylenol in Form eines 2,5-Xylenol enthaltenden Isomerengemisches, welches umfaßt :
Umsetzung des rohen 2,4-Xylenol-Gemisches mit einem Aldehyd in der 0,3- bis 3,0-fachen molaren Menge des 2,5-Xylenols in Gegenwart einer aromatischen Sulfonsäure, oder in Gegenwart einer aromatischen Sulfonsäure zusammen mit Wasser in einer solchen Menge, daß die Trennung in zwei Schichten aus Wasser und dem rohen 2,4-Xylenol unterbleibt,
Umwandlung des 2,5-Xylenols in ein Xylenol-Aldehyd-Kondensationsprodukt, und
Umwandlung der aromatischen Sulfonsäure in ein Ammoniumsalz derselben nach Beendigung der Umsetzung, sowie
anschließende Destillation der Reaktionsprodukte zur Abtrennung des 2,4-Xylenols durch Destillation.

## Revendications

1. Procédé de purification de 2,4-xylénol à partir d'un mélange brut d'isomères du 2,4-xylénol contenant du 2,5-xylénol, qui comprend les étapes consistant à faire réagir le mélange brut de 2,4-xylénol avec un aldéhyde, présent en une quantité comprise entre 0,3 et 3,0 fois en mole la quantité de 2,5-xylénol, en présence d'un acide sulfonique aromatique et ensuite à séparer du 2,4-xylénol le 2,5-xylénol sous forme d'un condensat xylénol-aldéhyde.

2. Procédé de purification de 2,4-xylénol à partir d'un mélange brut d'isomères du 2,4-xylénol contenant du 2,5-xylénol, qui comprend les étapes consistant à faire réagir le mélange brut de 2,4-xylénol avec un aldéhyde, présent en une quantité comprise entre 0,3 et 3,0 fois en mole la quantité de 2,5-xylénol, en présence d'un acide sulfonique aromatique tout en incorporant au système réactionnel de l'eau en une quantité telle qu'elle ne provoque pas la séparation de l'eau et du 2,4-xylénol brut en deux couches et ensuite à séparer du 2,4-xylénol le 2,5-xylénol sous forme d'un condensat xylénol-aldéhyde.

3. Procédé selon la revendication 1, dans lequel le mélange brut d'isomères du 2,4-xylénol utilisé comme produit de départ contient du 2,4-xylénol en une quantité comprise entre 80 % et 95 %.

4. Procédé selon la revendication 1, dans lequel la réaction entre l'aldéhyde et le 2,5-xylénol est mise en oeuvre à une température comprise entre 0 et 100°C.

5. Procédé selon la revendication 4, dans lequel la température est comprise entre 20 et 60°C.

6. Procédé selon la revendication 1, dans lequel la quantité d'acide sulfonique aromatique est comprise entre 0,001 et 10 parties en poids pour 100 parties en poids de 2,4-xylénol brut.

7. Procédé selon la revendication 6, dans lequel la quantité d'acide sulfonique aromatique est comprise entre 0,01 et 1 partie en poids pour 100 parties en poids de 2,4-xylénol brut.

8. Procédé selon la revendication 2, dans lequel la quantité d'eau incorporée dans le système réactionnel est comprise entre 2 et 20 parties en poids pour 100 parties en poids de 2,4-xylénol brut.

9. Procédé de purification de 2,4-xylénol à partir d'un mélange brut d'isomères du 2,4-xylénol contenant du 2,5-xylénol, qui comprend les étapes consistant à faire réagir le mélange brut de 2,4-xylénol avec un aldéhyde présent en une quantité de 0,3 à 3,0 fois en mole la quantité de 2,5-xylénol, en présence d'un acide sulfonique aromatique ou en présence d'un acide sulfonique aromatique coexistant avec de l'eau en une quantité telle qu'elle ne provoque pas de séparation en deux couches de l'eau et du 2,4-xylénol, à transformer le 2,5-xylénol en un condensat xylénol-aldéhyde et à transformer l'acide sulfonique aromatique en un sel d'ammonium de celui-ci après la fin de la réaction, et à distiller ultérieurement des produits de réaction afin de séparer le 2,4-xylénol par distillation.
